# EUROPEAN PATENT SPECIFICATION

(11) **EP 1 506 309 B1**
(45) Date of publication and mention of the grant of the patent: **22.11.2006**
(21) Application number: 03718589.9
(22) Date of filing: 14.05.2003
(51) Int. Cl.: C12Q 1/04, C12Q 1/44, C07H 11/04

(54) **PLATING MEDIA**
PLATTIERUNGSMEDIEN
MILIEUX DE MISE EN CULTURE

(30) Priority: 17.05.2002 US 147323
(43) Date of publication of application: 16.02.2005
(73) Proprietor: Biosynth AG, 9422 Staad (CH)
(72) Inventor: SCHABERT, Günter, CH-9403 Goldach (CH)
(74) Representative: Felder, Peter
(86) International application number: PCT/CH2003/000308
(87) International publication number: WO 2003/097856

(56) References cited:
- WO-A-99/48899
- MANAFI, M.: "Fluorogenic and chromogenic enzyme substrates in culture media and identification tests" INTERNATIONAL JOURNAL OF FOOD MICROBIOLOGY (1996), 31(1-3), 45-58 , 1996, XP002261071
- BRENNER K P ET AL: "NEW MEDIUM FOR THE SIMULTANEOUS DETECTION OF TOTAL COLIFORMS AND ESCHERICHIA COLI IN WATER" APPLIED AND ENVIRONMENTAL MICROBIOLOGY, WASHINGTON,DC, US, vol. 59, no. 11, 1 November 1993 (1993-11-01), pages 3534-3544, XP000539968 ISSN: 0099-2240

## Description

The present invention relates to plating media for detection and identification of various bacteria, yeasts or molds.

Potentially fluorogenic/chromogenic compounds are compounds which become fluorogenic/chromogenic and, hence fluoroscopically/visibly/spectroscopically detectable upon contact with certain microorganisms or substances produced by such microorganisms. Such compounds have been disclosed in WO 99/48899 (= EP 949266) and WO 98/38332, respectively, by applicant. In each case, it is either the fluorogenic or the chromogenic compound that is being used for detection.

As disclosed in these references, the enzyme termed "phosphatidylinositol-specific phospholipase C" (i.e. 1-phosphatidyl-D-myo-inositol inositolphosphohydrolase or PI-PLC for short herein; enzyme classification EC 3.1.4.10) can be found in culture supernatants of various bacteria including pathogenic bacteria such as *Listeria monocytogenes, Listeria ivanovii,* bacteria of the *Bacillus cereus group,* in particular *Bacillus cereus and Bacillus thuringiensis, Staphylococcus aureus* and *Clostridium novyi* (cf. J.G. Songer, Trends in Microbiology 5 (1997), 156).

A culture medium for detection of *Listeria monocytogenes* has been disclosed in WO 99/04032 consisting primarily of agar, nutrients as well as antibiotics to suppress unwanted germs and, again, makes use of only a chromogenic substrate.

By the same token, the plating medium for *Bacillus cereus* and *Bacillus thuringiensis* disclosed in US 6,284,517 makes use of only a chromogenic substrate of the kind noted above.

WO99/48899 describes the detection of PI-PLC producing microorganisms by the successive use of a fluorogenic and chromogenic agent. Manafi (International Journal of Food Microbiology (1996), vol. 31, pages 45-58) describes the simultaneous use of a fluorogenic and chromogenic agent for the detection of two different markers for detecting microorganisms in a media.

The major advantage in using PI-PLC substrates for microbiological diagnosis resides in the fact that PI-PLC enzymes are virulence factors of these microorganisms and such media serve well in food microbiology.

Yet a drawback of the media mentioned above is the time for carrying out the test. With the Listeria detection system disclosed in the above mentioned WO 99/48899 (EP 949266) it usually takes 24 hours to get an unequivocal fluoroscence in the selective enrichment broth, and it takes at least an additional time of from 24 up to 48 hours incubation of the plating media in order to grow the microorganisms to colonies showing a definite color. On the other hand, fluorogenic compounds when used in significant concentrations may decrease stability of the enrichment medium. Such substrates may also influence resuscitation efficacy and selectivity of the medium.

On the other hand, using a chromogenic plating medium alone means to renounce a quick screening tool for the microorganism of interest (the target microorganism). Additionally, as the primary sample has to be enriched anyway in order to grow the microorganisms to a sufficient cell density before streaking on a plating medium, time cannot be shortened considerably this way.

As it is a primary task, e.g. in food testing, not only to detect but to count the number of bacteria present in the sample, there is great demand for a quick and reliable method for identification and counting.

Yet many plating media mentioned above are not always selective enough for the microorganism of interest in that the growth of unwanted bacteria is not sufficiently suppressed. Furthermore the chromogenic response to bacterial PI-PLC is not optimized in order to get deeply colored colonies with a minimum amount of the chromogenic substrate.

A better differentiation of the pathogenic microorganisms producing PI-PLC from other species would be desirable, too.

Surprisingly it has now been found that a combination of both a chromogenic and a fluorogenic substrate, e.g. incorporated in a solid agar medium, may be used to both simultaneously screen as well as to detect pathogenic bacteria producing a phosphatidylinositol-specific phospholipase C.

Accordingly, the plating medium according to the present invention is as defined in claim 1. Preferred embodiments of the inventive plating medium are as specified in claims 2 - 27.

Further, the invention provides for a novel detection method as defined in claim 28 while preferred method embodiments are as specified in claims 29 - 31.

Generally, an essential advantage of plating media according to the invention resides in the time saved (e.g. at least 24 to 48 hours) if compared with prior art methods.

Plating media according to the invention provide improved means for screening as well as for detection, isolation and counting of bacteria, yeasts and molds which produce a PI-PLC enzyme, e.g. as a virulence factor such as *Listeria* spp. and *Bacillus* spp. In this context, selection of single nutrients as well as the selection of a combination of suitable nutrients by characterization of the growth parameters, e.g. by analysis using a Bioscreen C apparatus can be improved.

The terms "potentially fluorogenic" and "potentially chromogenic" as used herein with reference to the compounds mentioned above indicate the capacity of these compounds to become "fluorogenic" or "chromogenic", respectively, - i.e. fluoroscopically/visibly active and detectable by fluoroscopic methods and visual inspection or chromoscopy, respectively, upon interaction with PI-PLC .

Upon contact with PI-PLC, a potentially fluorogenic compound of formula (I) becomes a strong fluorophore which can be easily detected on the plating medium, e.g. by a conventional hand held fluorometer, e.g. for operation at a wavelength in the ultraviolet range which is favorable for practical purposes, such as 366 nm.

This simple detection approach can be used, according to the invention as a first screening step in the novel identification procedure. A fluorogenic compound for screening is advantageous because detection of a fluorophore is considerably more sensitive than detection of a chromophor. Accordingly, early notice of the presence of microorganisms secreting PI-PLC is now possible when use is made of a solid plating medium according to the invention.

In this screening test, lack of fluorescence can be considered a "negative" reaction in the sense that no further incubation of the medium is required whereas a "positive" reaction in the primary step needs to be verified by further incubation in order to develop a color.

Plating media according to the invention are suitable for detection, identification and enumeration of species of the *Bacillus cereus-group,* e.g. *Bacillus cereus, Bacillus thuringiensis* and *Bacillus anthracis* as well as for detection, identification and enumeration of *Listeria monocytogenes* and *Listeria ivanovii.*

The method according to the invention is also suitable for detection and identification of species or strains from the *Bacillus cereus-*group and enumeration of probiotic strains of *Bacillus cereus* (Paciflor^{®}, Toyocerin^{®}). Probiotic *B. cereus* are non-toxic "attenuated" strains. In contrast to the resistant *B. cereus* wild strains they are susceptible to penicillin G and cefamandole. Rapid recognition of probiotic versus wild strains is a significant advantage in *B. cereus* diagnosis.

The plating media of the present invention may also be used for direct counting of the target microorganisms without any enrichment step as well as for the so-called contact plate method, i.e. where the sample of interest is placed directly onto the medium for inoculation. This simple and fast test protocol is advantageous because no enrichment step is necessary. It is especially useful for food testing and hygienic control.

A neutralizing agent for eventually overcoming the inhibition by, e.g. disinfectants, occurring in such samples has to be added when the medium is used for direct plating. A further advantage of a neutralizing agent such as lecithin is its contribution to a better recognition of the target microorganisms by enhancing the coloration and by formation of a precipitation zone around the colonies of the target microorganism.

A particularly preferred compound for use as a fluorogenic substrate of formula (I) according to the invention is the N-methyl-morpholine salt of 4-methylumbelliferyl myo-inositol-1-phosphate.

In general, racemic mixtures of formula (I) and/or (IV) compounds are suitable for use in the plating media according to the invention.

For example, 4-methylumbelliferone (resulting from cleavage of the preferred 4-methylumbelliferyl myo-inositol-1-phosphate or a salt thereof by PI-PLC) has an absorption maximum of 360 nm at pH values above 8 whereas the corresponding formula (I) compounds show only a negligible absorption at 360 nm.

4-Methylumbelliferone (4-methyl-7-hydroxy-coumarin) is a very good fluorogen with an emission maximum at 448 nm (excitation at 364 nm).

Turning to detection methods according to the invention, the term "primary sample" used herein refers to the material obtained directly from a suspected source that may be of physiological or other origin, such as blood, excrements or infected foods, feeds, water sources, drinks or the like materials capable of harbouring the bacteria of interest. Also samples from food processings are of interest.

In both of its aspects as a screening and identification test, respectively, the invention provides for means to indicate bacterial activity of microorganisms having PI-PLC activity, including members of the *Bacillus cereus-group,* such as *Bacillus thuringiensis* and *Bacillus cereus* as well as *Listeria monocytogenes, Listeria ivanovii, Staphylococcus aureus, Clostridium novyi, Clostridium perfringens, Legionella* spp., *Helicobacter pylori, Trypanosoma brucei, Aspergillus fumingatus.*

The invention is of particular use for screening, detection and isolation of such pathogens as *Listeria monocytogenes, Bacillus cereus* and *Bacillus anthracis.*

*Bacillus anthracis* is unable to produce PI-PLC and can therefore be differentiated well from the other members of the *B. cereus group* by its typical but non-colored colonies.

When using compounds of formula (I) and/or (IV) in a screening and/or identification test for bacterial activity as evidenced by the presence of PI-PLC, it may be advantageous to provide an enrichment broth in which the primary sample is transferred in order to increase the bacterial activity prior to plating. It is preferred for many applications of the inventive method to use an enrichment broth which, in addition, may be selective for the bacteria of interest. The term "enrichment" which may be but need not be selective is understood by those experienced in the art who are capable of selecting an enrichment method that is most suitable for the bacteria of interest. Generally, inhibition of growth of other bacteria producing PI-PLC enzyme can be accomplished using various combinations of selective compounds including antibiotics and other inhibitors and the medium can be made specific for any pathogen that contains or produces PI-PLC.

Generally, the invention is believed to provide for some synergy of formula (I) if combined with formula (IV) compounds.

In a group of preferred compounds of formula (IV), R⁶, R⁷ and R⁸ are hydrogen or halogen atoms independently selected, preferably chlorine and bromine; while R⁹ and R¹⁰ are hydrogen and X is hydroxy; again, the salts of such compounds with organic or inorganic bases can be used as the potential chromophore, i.e. yielding deeply colored indigo dyes upon cleavage by PI-PLC, dimerization and subsequent oxidation, especially wherein R¹⁰ is hydrogen or methyl.

Particularly preferred compounds of formula (IV) for use as the chromogenic constituent are the salts of 5-bromo-4-chloro-3-indoxyl myo-inositol-1-phosphate, 5-bromo-6-chloro-3-indoxyl myo-inositol-1-phosphate and 6-chloro-3-indoxyl myo-inositol-1-phosphate. Among these, the ammonium salt of 5-bromo-4-chloro-3-indoxyl myo-inositol-1-phosphate is particularly preferred. Generally, formula (IV) compounds can be used in racemic form.

To illustrate an important embodiment of the present invention a preferred test method for identifying *Listeria monocytogenes* will now be explained in more detail. This method optionally comprises use of an enrichment broth that can repair or resuscitate injured *Listeria monocytogenes* cells according to EN ISO 11290.

Then, a small portion, e.g. a wire loop transporting a small amount of liquid of the enrichment broth, is streaked on a selective plating medium that contains the fluorogenic substrate, preferably a 4-methylumbelliferyl myo-inositol-1-phosphate of formula (I) as defined above.

Preferably, this plating medium is made selective as well to prevent growth of other bacteria, except *Listeria spp.,* containing the PI-PLC enzyme and *Listeria* related Gram-positive bacteria giving *Listeria monocytogenes* cells an optimal environment for growth on the plating medium producing isolated colonies.

Subsequent to incubation, typically for 16 to 24 hours, the medium is exposed to an UV fluorometer (long wavelength at 366 nm) and examined for fluorescence. A positive fluorogenic reaction around the small colonies grown up to this point indicates a presumptive positive test requiring further testing and no fluorescence means no *Listeria monocytogenes* is present in the sample tested.

After incubation for a further period of time and in the presence of, for example, *Listeria monocytogenes,* the plating medium containing a potentially chromogenic compound of formula (IV) will show colored colonies with the color developed by the chromogenic compound when in contact with PI-PLC. For example, with a substrate containing 5-bromo-4-chloro-3-indoxyl myo-inositol-1-phosphate a colony of *Listeria monocytogenes* will generate a turquoise to blue color. The colony can be isolated for further testing if required.

Generally, the chromogenic constituent works best on a solid surface since the color of the chromogen will be retained within the cell causing the color of the colony to be the color of the chromogen which is water insoluble and remains in the colony.

Plating media according to the invention tend to have good stability. For example, after 4 weeks of storage at 4°C in the dark, color and selectivity of the plating medium will be essentially the same as that of the freshly prepared medium. The *Listeria monocytogenes* colonies appear turquoise to blue and convex, 1.0-2.5 mm in diameter, without or with a turquoise to blue halo.

It is surprising that fluorogenic compounds of formula (I), e.g. in a plating medium containing 4-methylumbelliferyl myo-inositol-1-phosphate, are efficacious not only in a broth medium but also on a solid medium, such as agar.

Despite the fact that the fluorescence may leach from the colony into the agar medium, fluorescence serves as a screening test for presence or absence of pathogenic bacteria secreting PI-PLC enzymes.

Consequently, a positive reaction (secretion of the PI-PLC enzyme by a bacterial species) in the presence of the fluorogenic substrate will cause the colony to show fluorescence indicating that a PI-PLC secreting bacterial species is present at a significant cell density.

This is a presumptive positive reaction for the bacterial species of interest (such as *Listeria monocytogenes*) which warrant further isolation of the bacterial species in the form of a colony using the chromogenic substrate (IV) of the PI-PLC enzyme as explained above.

The fluorogenic formula (I) constituent and its fluorescence does not impair the enzymic color reaction of the chromogenic formula (IV) constituent of the combination or plating medium according to the invention.

Generally, presence of a fluorogenic constituent of formula (I) and of a chromogenic substrate of formula (IV) in a single medium allows for a fast presumptive positive reaction (due to the fluorogenic constituent) within a period of typically 24 hours followed by incubation for a further period of time, e.g. 24 hours, and identifying the colony on the solid medium using the chromogenic effect as a confirmatory reaction.

A further advantage of preferred plating media according to the invention resides in the generation of a white precipitation zone around, e.g. turquoise-colored colonies of *L. monocytogenes* /*L. ivanovii.* Optionally, the colony can be isolated for further testing.

As will become apparent from the following examples, the invention provides for novel and improved plating media for screening as well as for isolation, characterization, and quantitative evaluation (microbial count) of various hygienically and pathologically important microorganisms capable of metabolic production of a phosphatidylinositol-specific phospholipase C (PI-PLC) for which examples will be given below.

Typically, the formula (I) and (IV) constituents and notably the preferred compounds, such as 4-methylumbelliferyl-myo-inositol-1-phosphate can be used at a concentration of from about 0.05 - 0.3 grams per liter (g/l) while a typical concentration of 5-bromo-4-chloro-3-indoxyl myoinositol-1-phosphate will be in the range of from about 0.1 - 0.4 g/l. (The term "about" as used herein indicates a possible deviation of up to 50% of the value given).

While higher amounts might be used, no advantages compensating the higher costs will be normally obtained. It is to be emphasized that the concentration figures given here and in the following examples are based upon the complete ready-to-use medium. For producing dry plating media (e.g. for increased storage life or other forms of application) proper amounts must be calculated accordingly.

Substituents R¹ through R¹⁰ in formula (I) and (IV) are fluorogenic or chromogenic, respectively, in the sense that such substituents will increase or, at least, not significantly diminish the specific absorption of light of the formula (I) and/or (IV) compounds that produces fluorescence and/or color, respectively, upon exposure to PI-PLC.

Generally, plating media compositions are well known per se in the art (cf., for example, "Biotechnological Bioengineering" Vol. 24, 1982, pp 1519) and are available as liquid, semi-liquid, or solid culture media, generally containing:
- a gel-forming constituent, such as agar or gelatin;
- nutrients including a carbon source for the microorganisms of interest,
- various additives, and (depending upon the desired form)
- an optional aqueous medium as required by the gel-forming capacity of the gel-forming constituent.

As will be apparent to those skilled in the art, a carrier or substrate, such as typically a petri dish, is used as a support for the liquid or semi-liquid plating medium.

Plating media according to the invention include such liquid or semi-liquid forms as well as dry plating media. A preferred "dry" is substantially anhydrous, typically containing less than 5 %, by weight, and preferably less than 3 %, by weight, of water for storability. In other words, a typical dry or anhydrous plating medium according to the invention may be constituted such that it will yield a liquid or semi-liquid plating medium upon addition of sufficient aqueous medium.

Preparation of plating media follows the well known rules, e.g. as described by the manufacturers of the conventional constituents.

It is to be noted that the term "ready-to-use" generally includes plating media of various consistencies ranging from a maximal to a minimal water content of the plating medium used for culturing, and includes "aqueous" or "liquid" as well as "semi-dry" or "semi-liquid" plating media. As is well known in the art of microbial culturing, a typical plating medium is made up as a pourable or "liquid" composition (sometimes termed a "sol") which will form a relatively firm gel after being applied to a carrier, such as a petri dish or other type of supporting plate. Thus, plating media according to the invention in their ready-to-use liquid or semi-liquid form may, but need not, include such a carrier or plate.

A typical anhydrous plating medium according to the invention will include all basic ingredients required for the intended use, i.e. the gel-forming constituent, the formula (I) and/or formula (IV) compound and - in general - essential nutrients for microbes, and a preferred anhydrous plating medium according to the invention will include all essential constituents of the ready-to-use plating medium except the aqueous constituent, e.g. in the form of a freeze-dried composition or lyophilisate.

It should be noted however that the essential characteristic of a plating medium according to the invention is that fact that it contains at least one formula (I) and formula (IV) compound for detection of microbial PI-PLC by fluorescence and color formation. Accordingly, nutrients, specific additives for selectivity (inhibitors) or activators for growth promotion, improved enzymatic cleavage, or additives for use in contact plate methods may - and preferably are - but need not be contained in a dry, semi-liquid (synonymous with semi-solid) or liquid plating medium according to the invention since the actual user may wish to adapt it in view of specific requirements.

Among various preferred applications of plating media according to the invention is their use for the so-called contact plate method just mentioned, i.e. where the sample of interest, e.g. a food product such as cheese, or a tool or apparatus component used in food processing (hygienic monitoring), is placed directly onto the medium on the plate for inoculation and hygienic control. The plating medium for this method preferably contains a component capable of counteracting surface-active substances that may be contained in typical samples. Phospholipids, such as lecithin, and L-histidine are suitable additives for this and other purposes as explained below in more detail. Typical commercial contact plates are of rectangular shape (20 by 80 mm) and are filled to the extent of forming a slight convex outward bulge for good contacting properties.

Semi-liquid media can be inoculated and then applied to a non-specific nutrient layer, or used for migration of the microorganisms of interest due to their mobility and/or under the impact of an electric field. Fluorescence and/or color formation will be observed in the semi-solid portion of the plating medium.

Gelling agents (also termed gel forming agents) for use with plating media are well known in the art and are available commercially. Suitable agents, such as various types of agars or gelatine for use in microbial cultures including plating media according to the invention are capable of forming an aqueous gel.

Preferred nutrients for all types of plating media according to the invention include mixtures of peptidic, pancreatinic, tryptic and papainic peptones from casein, soy, meat and mixtures of amino acids, e.g. casamino acids, notably with the aim to shorten the lag phase of microbial growth. Peptone mixtures can be replaced by high-quality nutrient agar, such as Columbia agar.

Preferred mixtures of amino acids are those containing L-cysteine und L-tryptophane and are used typically in amounts of about 10 - 200 milligrams per liter (mg/l), calculated for the final or ready-to-use plating medium.

Additives found to be suitable for plating media according to the invention are essential growth agents for the microbes of interest including vitamins, e.g. in the form of yeast extract or meat extract (the term "meat" including, inter alia, organs such as liver, blood constituents) and brain-heart-infusion, typically in an amount of about 1 to 12 g/l.

Carbon sources found to be suitable for plating media according to the invention include, inter alia, carbohydrates or their metabolic precursors, e.g. D-glucose, sodium pyruvate, and L-rhamnose, typically at a concentration of from about 0.5 to 5 g/l. Sodium pyruvate also reduces the amount of oxygen radicals acting inhibitory for growing cells.

To improve growth and PI-PLC cleavage, trace elements can be added, for example magnesium salts, e.g. MgSO₄, and the standard trace element solution "Schlösser" (cf. W. Dunger, H.J. Fiedler; Methoden der Bodenkunde, 2nd edition, 1997, pp 92, Gustav Fischer, Jena-Stuttgart/Germany) in typical concentrations of from about 0.2 -1.0 g/l. It has been found that such a trace element solution will enhance PI-PLC production and, thus, cleavage of the formula (I) and/or (IV) compound with the result of generating fluorescence and/or coloration in a substantial and unexpected degree.

Another preferred additive is a source of ferric or ferrous ions, e.g. ferric citrates, such as ferric ammonium citrate (e.g. at a concentration of about 0.1 - 1.0 g/l), or ferrioxamines, such as ferrioxamin B, e.g. at a concentration of about 50 - 2000 micrograms per liter (µg/l), preferably 100 - 500 µg/l.

To promote the cleavage action of PI-PLC, a serum, e.g. horse or bovine serum, can be added, typically at a concentration of about 20 - 100 ml/l, or albumin from bovine serum, typically at a concentration of about 2 - 5 g/l. Use of such albumin is of particular advantage when producing a dry plating medium.

Another activator or promoter for PI-PLC are phospholipids, e.g. lecithin from soy beans, other semen or egg yolk, typically at a concentration of about 1-5 g/l, and salts of glycero phosphoric acid, e.g. magnesium glycero phosphate or sodium glycero phosphate, at a concentration of about 0.5 to 2.0 g/l. Use of such constituents is of notable importance in plating media for use in the contact plating method mentioned briefly above. L-histidine, e.g. used in a typical concentration of about 0.5 to 2 g/l, is another additive for use in plating media for direct contact.

An inert opacifier, such as titanium dioxide, typically in an amount of about 1-5 g/l, may be added to improve recognition of colored colonies.

Natural starch, e.g. at a concentration of about 1 - 5 g/l, can be added to reduce the size of the colonies and to improve efficiency of the fluorogenic and/or chromogenic indicators used in plating media according to the invention.

It is advantageous to make the plating medium selective for specific microorganisms of interest. For example, in order to provide selectivity for *Listeria monocytogenes,* growth of other PI-PLC producing microorganisms, such as other Gram-negative or Gram-positive bacteria , e.g. *Bacillus cereus,* as well as yeasts and molds is disadvantageous and should be inhibited to prevent confusion in reading the plates.

On the other hand, if the plating medium is to be made selective for microorganisms other than *Listeria,* suitable inhibitors will have to be selected accordingly. Thus, the inhibitors mentioned below are given by way of an illustrative example for plating media which are selective for *Listeria.* Those experienced in the art can easily select suitable inhibitors on the basis of general microbiological knowledge and/or with a few and simple growth experiments.

Suitable inhibitors for *Listeria-specific* plating media include combinations of antibiotics working synergistically, e.g. a combination of antibiotics such as polymyxin B, e.g. at a concentration of about 10 to 20 milligram per liter (mg / l), optionally combined with sulfamethoxazole (e.g. at about 50 - 1000 mg / l) as well as phosphomycin (e.g. at about 20 - 50 mg / l) or ceftazidime (e.g. at about 20 - 50 mg / l), doxycycline (e.g. at about 2-20 mg / l) for inhibiting Gram-negative bacteria.

Further, addition of lithium chloride (e.g. at about 2 - 20 g/l), preferably in combination with nalidixic acid (e.g. at about 20 - 50 mg /l) or clindamycin (e.g. at 1-10 mg / 1), serves to suppress unwanted Gram-positive bacteria.

Inhibition of yeasts and molds can suitably be accomplished by addition of cycloheximide (e.g. at about 50 - 300 mg /l) or amphotericin B (e.g. at about 1 - 5 mg /l).

Generally, such mechanisms apply to media for detection and count determination of other microorganisms producing PI-PLC, notably *Bacillus cereus* and *Bacillus thuringiensis* and selectivity can be achieved in an analogous manner as explained above for *Listeria.*

Inhibition for *Bacillus cereus-*group specific plating media is achieved, according to a preferred mode of operation, by using a combination of antibiotics acting synergistically, e.g. a combination of polymyxin B (10 - 20 mg / l), sulfamethoxazole (10 - 200 mg / l) and trimethoprim (1 - 20 mg / l).

The *Bacillus cereus-group* specific plating medium disclosed in the present invention may also be used for characterization and differentiation of so-called "probiotic" *Bacillus cereus* isolates from animal feeds. The non-toxic "attenuated" *Bacillus cereus* strains Paciflor^{®} and Toyocerin^{®} are used as feed additives acting as fertilizers.

The differentiation of these probiotic, spore-forming products from toxic *Bacillus cereus* wild strains cannot be done by simple, e.g. biochemical tests as these strains behaved equal in hemolysin-production, starch hydrolysis, catalase as well as gelatinase and lecithinase reaction and do not show any significant differences in fermentation of carbohydrates.

However, the non-toxic probiotic *Bacillus cereus* strains noted above unexpectedly showed delayed production of PI-PLC detectable on the new *Bacillus cereus-group* plating medium according to the invention by substantial weaker colorization (e.g. light turquoise colonies).

In order to get an unequivocal differentiation from weak PI-PLC producing wild-type strains of *Bacillus cereus,* antibiotic discs may be applicated onto the surface of the medium. Whereas *B. cereus* wild strains behaved resistant to Penicillin G (10 µg/disc) and cefamandole (30µg/disc) all tested probiotic *B. cereus* strains were susceptible.

Non-limiting examples of microorganisms for detection and count by means of plate media according to the invention include members of the *Bacillus cereus-group,* notably *Bacillus cereus, Bacillus thuringiensis, Bacillus mycoides, Bacillus weihen-stephanensis* and *Bacillus anthracis* as well as *Listeria monocytogenes, Listeria ivanovii, Staphylococcus aureus, Legionella pneumophila, Clostridium species, Helicobacter pylori.* Among further microbial organisms of interest are yeasts, e.g. *Candida species* and molds, e.g. *Aspergillus species.*

A preferred test method for identifying *Listeria monocytogenes* comprises use of an enrichment broth as described above that can repair or resuscitate injured *Listeria monocytogenes* cells, e.g. a combination of a so-called Fraser ½-broth for pre-enrichment followed by selective enrichment with normal Fraser-broth as described in EN ISO 11290-1 and EN ISO 11290-2.

Then, the plating medium is inoculated by a small portion, at least, of the enrichment broth, e.g. by means of a wire loop for transporting a small amount of liquid. Since the plating medium contains a fluorogenic and/or chromogenic compound; i.e. exhibit fluorescence or coloring upon contact with PI-PLC produced by the microorganism of interest thus indicating presence of microorganisms of interest, and - because of the selective growth conditions provided by the composition of the plating medium - incubation of the plating medium leads to growth of the microorganisms of interest and to formation of corresponding microbial colonies exhibiting fluorescence and/or coloration. If desired, microbial cells can be isolated from the fluorescent and/or colored colonies for verification.

The plating medium may also directly be inoculated with the samples, e.g. with various food-samples for direct counting the pathogens.

The contact plate method mentioned above and the direct-plate medium according to the invention made selective as disclosed above is of particular interest for control and monitoring purposes in the food-processing industries. The following non-limiting examples are given to further illustrate the invention.

Generally, the invention provides for safe, sensitive and commercially viable detection, identification and counting of potentially pathogenic bacterial activity of such microbes as *Listeria monocytogenes, Listeria ivanovii, Bacillus cereus* including the probiotic *B. cereus -* strains (Paciflor^{®}, Toyocerin^{®}), *Bacillus thuringiensis* and *Bacillus anthracis* in potentially infected materials including physiological samples or consumable goods such as foods, beverages and animal feeds.

The invention will now be explained in more detail by way of non-limiting

### examples.

### EXAMPLES

### Examples 1-3: Improved selective plating media for Listeria monocytogenes

### Example 1:

This example illustrates preparation, inoculation and efficacy of an improved ready-to-use and selective plating medium for *Listeria monocytogenes* with a fluorescent compound of formula (I) according to the invention.

Plating agar was prepared from the following basic ingredients:

| | |
|---|---|
| Agar | 15.0 g / l |
| Special peptone | 18.0 g / l |
| Yeast extract | 8.0 g / l |
| Casamino acids | 5.0 g / l |
| Meat extract | 5.0 g / l |
| Sodium glycerophosphate | 1.0 g / l |
| Sodium pyruvate | 1.0 g / l |
| Magnesium sulfate heptahydrate | 1.5 g / l |
| Lithium chloride | 5.0 g / l |
| Ferric ammonium citrate | 0.2 g / l |
| Potassium phosphate, dibasic (KH₂PO₄) | 1.5 g / l |
| Sodium phosphate, dibasic (NaH₂PO₄ • 2 H₂O) | 3.1 g / l |
| Cycloheximide | 0.2 g / l |

All ingredients were dissolved in 960 ml of water (distilled or de-ionized) and autoclaved at 121 °C for 15 minutes. The pH was maintained in the range of 7,1 - 7,2.

The following supplements were added in the amounts specified:

| | |
|---|---|
| D-Glucose | 2.0 g / l |
| Bovine serum albumin | 3.5 g / l |
| Soy lecithin | 1.5 g / l |
| Standard trace element solution Schlösser | 5 ml / l |
| 4-Methylumbelliferyl myo-inositol-1-phosphate, N-methylmorpholine salt | 0.1 g / l |

The following antibiotics were added as inhibitors for undesired microorganisms in view of selectivity for *Listeria* as explained above:

| | |
|---|---|
| Polymyxin B | 0.01 g / l |
| Nalidixic acid | 0.03 g / l |
| Ceftazidime | 0,03 g / l |

Bovine albumin was dissolved separately in 10 ml of water (distilled or de-ionized). Lecithin was suspended in 10 ml of 20 vol.% ethanol. Both solutions as well as the glucose, the solution of trace elements and the substrate were added aseptically while stirring to above plating agar after cooling to 50-55°C. Finally the antibiotics are added aseptically.

After stirring for ten minutes, the pH was controlled and the medium was poured into petri dishes and allowed to solidify.

The standard trace element solution "Schlösser" is composed of:

| | |
|---|---|
| Zinc sulfate heptahydrate | 1 mg / l |
| Manganese sulfate tetrahydrate | 2 mg / l |
| Boric acid | 10 mg / l |
| Cobalt(II) nitrate hexahydrate | 1 mg / l |
| Sodium molybdate dihydrate | 1 mg / l |
| Copper(II) sulfate pentahydrate | 0.005 mg / l |
| Ferrous sulfate heptahydrate | 0.7 g / l |
| EDTA | 0.8 g / l |
| Bi-distilled water | to 1 liter total volume. |

This standard Schlösser solution is prepared as a stock solution in ten-fold concentration and autoclaved at 121 °C for 15 minutes. 5 ml of this concentrated solution were added to 1 liter of the above plating medium.

The plating medium was inoculated by using a loop full of liquid from a preenrichment broth and streaking onto the plating medium, and incubated at 36 ± 1 °C for 24 hours. After first reading the plates were incubated for further 20 - 24 h at 36 ± 1 °C.

Results are summarized in Table 1 showing the colonial characteristics of a variety of bacteria on the selective/ differential plating medium after incubation.

### Example 2:

Example 1 was repeated except that 0.2 g/l of 5-bromo-4-chloro-3-indoxyl-myo-inositol-1-phosphate ammonium salt were added to provide a combination of both a fluorogenic (formula I) and a chromogenic (formula IV) indicator according to the invention.

Again, the results are summarized in Table 1.

### Example 3:

Example 1 was repeated except that the 0.1 g/l of 4-methylumbelliferyl-myo-inositol-1-phosphate N-methyl-morpholine salt (compound of formula I) were replaced by 0.2 g/l of 5-bromo-4-chloro-3-indoxyl-myo-inositol-1-phosphate-ammonium salt (compound of formula IV).

The turquoise to blue color of the *Listeria monocytogenes* colonies indicated the presence of the PI-PLC enzyme by forming the deeply colored 5,5'-dibromo-4,4'-dichloro-indigo water insoluble dye which was retained within the colony and did not diffuse into the medium.

Again, the results are summarized in Table 1.

**Table 1**

| Colonial morphologies of various bacteria on selective plating medium for *Listeria monocytogenes* obtained according to Examples 1 - 3 | |
|---|---|
| *Listeria monocytogenes* NCTC 7973 | Fluorescent and/or colored (blue to turquoise) flat colonies up to 2 mm in diameter with a white preciptation zone around the colony |
| *Listeria monocytogenes* 3208/99 | Fluorescent and/or colored (blue to turquoise) flat colonies up to 2 mm in diameter with a white precipitation zone around the colony |
| *Listeria ivanovii* DSM 20751 | Fluorescent and/or colored (blue to turquoise) flat colonies up to 2 mm in diameter with a white precipitation zone around the colony |
| *Listeria innocua* SV6A | Non-fluorescent and/or white colonies, up to 2 mm in diameter |
| *Escherichia coli* NCTC 10481 | No growth |
| *Pseudomonas aeruginosa* NM 17 | No growth |
| *Enterococcus faecalis* ATCC 33186 | Strongly reduced growth |
| *Bacillus cereus* ATCC 11778 | No growth |
| *Staphylococcus aureus* NCTC 6571 | No growth |
| *Staphylococcus epidermidis* CCM 2243 | No growth |
| *Candida albicans* 1695 | Strongly reduced growth |
| *Saccharomyces cerevisiae* 1688 | Strongly reduced growth |

### Examples 4-6: Improved selective plating media for Bacillus species

Selective fluorescent and/or chromogenic plating media for various *Bacillus* species were prepared essentially as described in Example 1 except as specified in Table 2 below. The plating medium of example 4 (comparative) contained the fluorogenic indicator (A). The plating medium of Example 5 contained both fluorogenic and chromogenic indicator (A + B) while the plating medium of Example 6 (comparative) contained but the chromogenic indicator (B). The composition of the nutrients is chosen in order to get colonies not to big since the amount of the fluorogenic / chromogenic substrate could be reduced.

**Table 2**

| **Composition/Basic Medium** | |
|---|---|
| | |
| Agar | 14.0 g/l |
| Proteose Peptone | 5.0 g /l |
| Tryptone | 5.0 g /l |
| Meat peptone, pancreatic | 5.0 g /l |
| Meat extract | 3.0 g /l |
| LiCl | 2.0 g /l |
| K₂HPO₄ | 4.0 g /l |
| pH 7.2-7.3 | |
| | |

| **Supplements:** | |
|---|---|
| Bovine serum albumin | 3.5 g / l |
| (A) 4-methylumbelliferyl myo-inositol-1-phosphate, N-methylmorpholine salt (Formula I compound) | 0.1 g / l |
| (B) 5-bromo-4-chloro-3-indoxyl myo-inositol-1-phosphate, ammonium salt (Formula IV compound) | 0.2 g / I |
| Trimethoprim | 0.0032 g / l |
| Polymyxin B | 0.020 g / l |
| Sulfamethoxazol | 0.016 g / I |
| Cycloheximide | 0.2 g / l |

The basic medium was autoclaved at 121 °C for 15 minutes and the pH was controlled to 7.1 - 7.3.

The supplements were dissolved aseptically in sterile distilled or de-ionized water and added aseptically to the plating medium after cooling to approximately 50°C. The complete medium was poured into petri dishes and allowed to solidify.

The plating medium was inoculated either by using a loopful liquid from a preenrichment broth and streaking onto the agar or directly by the sample. It was incubated at 36 ± 1 °C for 24 hours. Table 3 summarizes colony characteristics of a variety of bacteria on the improved selective/differential plating medium for *Bacillus* species after incubation.

A further advantageous mode of use of this medium is the detection of probiotic *Bacillus cereus* strains (Paciflor^{®}, Toyocerin^{®}) by application of antibiotic discs onto the surface of the medium shown in Table 2. The method performed is like the agar-diffusion test. Whereas *B. cereus* wild strains behaved resistant to penicillin G (10 µg/disc) and cefamandole (30 µg/disc) all tested probiotic *Bacillus cereus* strains were susceptible towards these antibiotics.

**Table 3**

| Morphologies of colonies of various bacteria on plating medium selective for *Bacillus spp.* obtained according to Examples 4 - 6 | | |
|---|---|---|
| Species | Number of strains tested | colonial morphology |
| *Bacillus anthracis* (wild type) | 16 | Non-fluorescent and/or un-colored large dull colonies up to 2-4 mm in diameter |
| *Bacillus cereus* | 19, among them ATCC 11778 | Fluorescent and/or colored (turquoise) large dull colonies up to 2-4 mm in diameter, with or without fluorescent and/or colored (turquoise) halos |
| *Bacillus cereus, probiotic strains* Paciflor^{®} and Toyocerin^{®} | 10 each | Week fluorescent and/or light-turquoise dull colonies up to 2-4 mm in diameter |
| *Bacillus thurin-giensis* | 4, among them ATCC 10792 | Fluorescent and/or colored (turquoise) large dull colonies up to 2- 4 mm in diameter, with or without fluorescent and/or colored (turquoise) halos |
| *Bacillus mycoides* | 1 | Fluorescent and/or light-turquoise, irregular edged colonies, 3-6 mm in diameter |
| *Bacillus weihen- stephanensis* | 4 | Fluorescent and /or turquoise colonies, 1-2 mm in diameter |
| *Bacillus circulans, Bacillus licheni-formis* | 1 strain each | No growth or small, nonfluorescent, un-colored colonies up to 2 mm |

| *Bacillus subtilis* ATCC 6633; *Bacillus lentus, B. megaterium, Bacillus pumilus* | 1 strain each | No growth |
|---|---|---|
| *Listeria monocyto- genes* | 4, among them NCTC 7973 | Small fluorescent and/or colored (turquoise) colonies; < 1 mm in diameter |
| *Listeria innocua, Listeria seeligeri, Listeria welshimeri* | 1 strain each | Small nonfluorescent, un-colored colonies up to 1 mm |
| *Enterococcus faecalis* ATCC 19433 | 1 | No growth |
| *Staphylococcus aureus* NCTC 6571 | 1 | No growth |
| *Escherichia coli* ATCC 25929 | 1 | No growth |
| *Salmonella typhimurium* ATCC 14028 | 1 | No growth |

It should be noted that while the above examples are concerned with preferred constituents 4-methylumbelliferyl myo-inositol-1-phosphate, N-methylmorpholine salt and 5-bromo-4-chloro-3-indoxyl myo-inositol-1-phosphate, ammonium salt, respectively, it is apparent from the general disclosures above that very similar results will be obtained with other substrates of formula (I) and (IV), respectively. Thus, various modifications of the examples given above will be apparent.

## Claims

1. A plating medium for detection of a microorganism of the type capable of metabolic production of a phosphatidylinositol-specific phospholipase C (PI-PLC) **characterized in that** said plating medium contains, in combination, at least one fluorogenic compound capable of generating fluorescence when in contact with said PI-PLC, and at least one chromogenic compound capable of producing a color when in contact with said PI-PLC; wherein said fluorogenic compound is represented by formula (I) 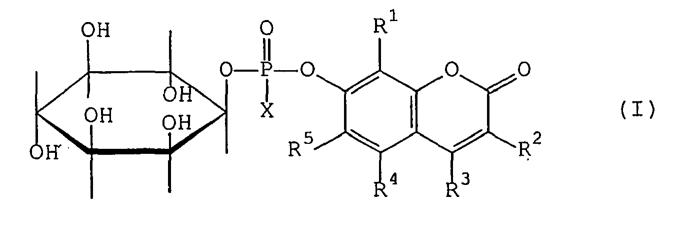 in which R¹,R²,R³,R⁴ and R⁵ are independently selected from the group consisting of hydrogen and fluorogenic substituents, and X is selected from the group consisting of: hydroxyl; OR^{y} wherein R^{y} is selected from the group consisting of C₁ - C₄ alkyl; and O⁻ Me⁺ wherein Me⁺ is a cation derived from an organic or inorganic base,
and wherein said chromogenic compound is represented by formula (IV) 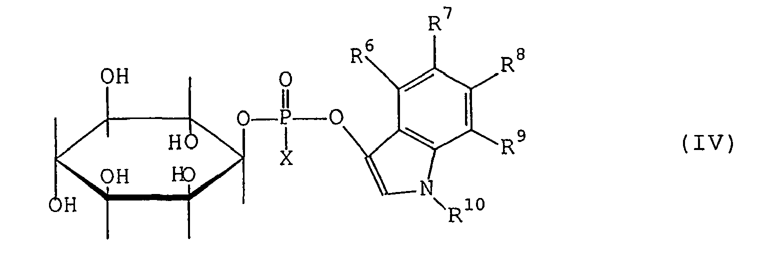 in which R¹⁰ is selected from the group consisting of hydrogen and C₁₋₄ alkyl and R⁶, R⁷, R⁸, and R⁹ are selected from the group consisting of hydrogen and chromogenic substituents; and X is as defined above.

2. The plating medium of claim 1 wherein said Me⁺ in Formula (I) and (IV) is a cation derived from sodium hydroxide, potassium hydroxide, lithium hydroxide, ammonium hydroxide, diethylamine, triethylamine, tetramethylammonium hydroxide, tetraethylammonium hydroxide, cyclohexylamine, pyridine, piperidine, pyrrolidine, morpholine, N-methyl-morpholine, N-ethyl-morpholine and p-toluidine.

3. The plating medium of claim 1 or 2 wherein each of said fluorogenic substituents in Formula (I) is independently selected from the group consisting of C₁-C₄ alkyl optionally containing an oxygen atom in the alkyl chain; C₁-C₄ alkoxy; nitro; carboxy, C₁-C₄ carboxyalkyl, and cyano, wherein any of said alkyl groups optionally includes at least one halogen atom as a substituent.

4. The plating medium of claim 3 wherein R³ in Formula (I) is C₁-C₄ alkyl optionally containing one or more halogen atoms, X is hydroxyl, and R¹,R²,R⁴ and R⁵ are hydrogen atoms; and wherein said salt of said compound of formula (I) is a salt formed with an organic or inorganic base.

5. The plating medium of any of claims 1 - 4 wherein said formula (I) compound is selected from 4-methylumbelliferyl myo-inositol-1-phosphate and salts thereof with an organic or inorganic base.

6. The plating medium of any of claims 1 - 5 wherein R⁶, R⁷ and R⁸ in said formula (IV) are selected from hydrogen, chlorine and bromine; R⁹ and R¹⁰ are hydrogen and/or wherein said formula (IV) compound is in the form of a salt with an organic or inorganic base.

7. The plating medium of any of claims 1 - 6 wherein said compound of formula (IV) is selected from 5-bromo-4-chloro-3-indoxyl myo-inositol-1-phosphate, 5-bromo-6-chloro-3-indoxyl myo-inositol-1-phosphate, 6-chloro-3-indoxyl myo-inositol-1-phosphate and a salt thereof.

8. The plating medium of claim 7 wherein said salt is an ammonium salt of said formula (IV) compound.

9. The plating medium of any of claims 1 - 8 additionally containing.at least one gel-forming constituent and at least one nutrient capable of supporting growth of said microorganism.

10. The plating medium of any of claims 1 - 9 additionally containing at least one inhibitor for microorganisms other than said microorganism of interest.

11. The plating medium of claim 9 or 10 wherein said nutrient is selected from the group consisting of peptones and amino acids.

12. The plating medium of claim 11 wherein said amino acids are used as acidically hydrolyzed casein in an amount of from about 2 to about 20 grams per liter of said medium with addition of L-cysteine and L-tryptophan in an amount of from about 10 to about 200 milligram per liter.

13. The plating medium of any of claims 9 - 12 wherein said nutrients are selected by nutrient analysis and whereby said nutrient includes at least one member of the group consisting of yeast extract, meat extract and brain heart infusion in an amount of from about 1 to about 12 grams per liter of said medium.

14. The plating medium of any of claims 1 - 13 additionally comprising at least one carbohydrate selected from the group consisting of D-glucose, sodium pyruvate, and L-rhamnose in an amount of from about 0.5 to about 5 grams per 1 of said medium.

15. The plating medium of any of claims 1 - 14 additionally comprising trace elements capable of improving growth of said microorganism.

16. The plating medium of any of claims 1 - 15 additionally comprising at least one ferric compound selected from the group of ferric citrate, ferric ammonium citrate, and ferrioxamines.

17. The plating medium of any of claims 1 - 16 additionally comprising a promoter for cleavage of PI-PLC selected from the group consisting of albumines, phospholipoids, and glycerophosphoric acids.

18. The plating medium of any of claims 1 - 17 additionally comprising an inducer for enzyme production, e.g. lecithin, wherein cleavage of said inducer contributes to a better recognition of the target microorganism by improvement of colony-coloration and by forming a white precipitation zone surrounding the fluorescent and/or colored colonies.

19. The plating medium of any of claims 1 - 18 additionally comprising a contrasting agent to improve visual detectability of dyed colonies of said microorganism of interest.

20. The plating medium of any of claims 10 - 19 wherein said inhibitor is selected from the group consisting of compounds capable of inhibiting growth of Gram-negative bacteria, Gram-positive bacteria, yeasts and fungi.

21. The plating medium of any of claims 10 - 20 wherein said inhibitor is selected from polymyxine, sulfamethazole, sulfamethoxazole, phosphomycin, doxycyclin, ceftazidime, clindamycin, nalidixic acid, cycloheximide, trimethoprim, and amphotericine.

22. The plating medium of any of claims 1 - 21 for use in a direct-contact method by direct contact with a sample suspected of containing said microorganism of interest comprising at least one constituent for counteracting surface active components of a sample.

23. The plating medium of claim 22 wherein said constituent for counteracting surface active components of a sample comprises lecithin and L-histidine.

24. The plating medium of any of claims 1 - 23 containing an aqueous medium for providing a ready-to-use plating medium.

25. The plating medium of any of claims 1 - 23 in a substantially anhydrous form.

26. The plating medium of any of claims 1 - 25 whereby selected antibiotica discs are put onto the surface of that plating medium for further differentiation of growing bacteria by their different resistance.

27. The plating medium of any of claims 1 - 26 wherein said microorganism to be detected is selected from *Listeria monocytogenes, Listeria ivanovii, Bacillus cereus,* probiotic strains of *Bacillus cereus, Bacillus thuringiensis, Bacillus mycoides, Bacillus anthracis, Staphylococcus aureus, Legionella pneumophila, Clostridium species, Helicobacter pylori, Candida species; and Aspergillus species.*

28. A method for detection of a microorganism of interest, said microorganism being of the type capable of metabolic production of a phosphatidylinositol-specific phospholipase C (PI-PLC); comprising the steps of:
(a) providing a plating medium containing an aqueous gel and at least one nutrient capable of supporting growth of said microorganism; and at least one compound of formula (I) as defined in claim 1 and at least one compound of formula (IV) as defined in claim 1;
(b) inoculating said plating medium with a sample material suspected of containing said microorganism of interest;
(c) incubating said plating medium subsequent to said step (b);
(d) optionally putting selected antibiotica discs onto the surface of that plating medium for further differentiation of growing bacteria by their different resistance; and
(e) observing said plating medium subsequent to said step (c) or (d) for formation of fluorescent and/or colored colonies of said microorganism of interest indicative of a presence of said microorganism in said sample.

29. The method of claim 28 wherein said compound of formula (I) is 4-methylumbelliferyl myo-inositol-1-phosphate or a salt thereof.

30. The method of claim 28 or 29 wherein said compound of formula (IV) is 5-bromo-4-chloro-3-indoxyl myo-inositol-1-phosphate or a salt thereof.

31. A combination of at least one formula (I) compound as defined in claim 1 and at least one formula (IV) compound as defined in claim 1 for use in the detection of microorganisms of the type capable of metabolic production of a phosphatidylinositol-specific phospholipase C (PI-PLC).

## Patentansprüche

1. Plattierungsmedium zum Nachweis eines Mikroorganismus eines Typs, der zur metabolischen Produktion einer phosphatidylinositol-spezifischen Phospholipase C (PI-PLC) fähig ist, **dadurch gekennzeichnet, dass** besagtes Plattierungsmedium in Kombination enthält: mindestens eine fluorogene Verbindung, die bei Kontakt mit besagtem PI-PLC zur Erzeugung von Fluoreszenz fähig ist, und mindestens eine chromogene Verbindung, die bei Kontakt mit besagtem PI-PLC zur Bildung einer Farbe fähig ist; wobei besagte fluorogene Verbindung durch die Formel (I) dargestellt ist, 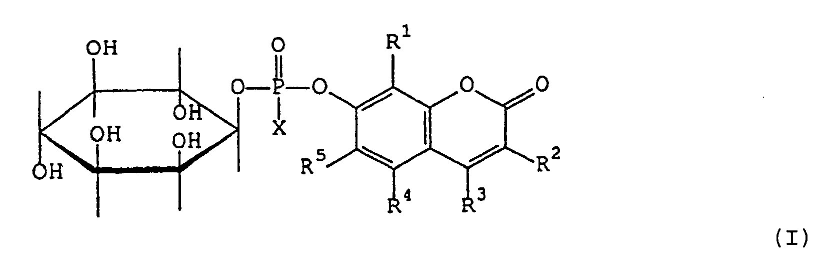 wobei R¹, R², R³, R⁴ und R⁵ unabhängig ausgewählt sind aus der Gruppe bestehend aus Wasserstoff und fluorogenen Substituenten, und X ausgewählt ist aus der Gruppe bestehend aus: Hydroxyl; OR^{Y}, wobei R^{Y} ausgewählt ist aus der Gruppe bestehend aus C₁-C₄₋Alkyl; und O-Me⁺, wobei Me⁺ ein von einer organischen oder anorganischen Base abgeleitetes Kation ist, und wobei besagte chromogene Verbindung durch die Formel (IV) dargestellt ist, 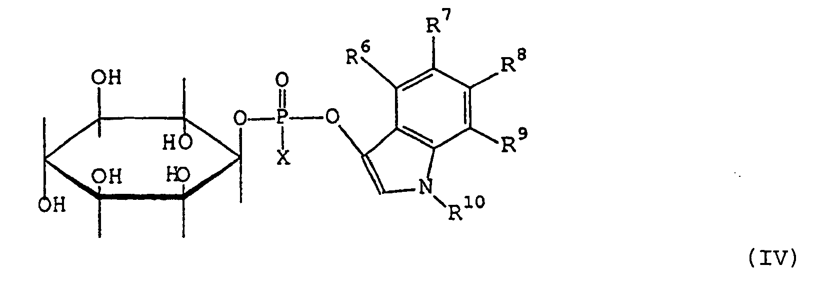 wobei R¹⁰ ausgewählt ist aus der Gruppe bestehend aus Wasserstoff und C₁₋₄-Alkyl und R⁶, R⁷, R⁸ und R⁹ ausgewählt sind aus der Gruppe bestehend aus Wasserstoff und chromogenen Substituenten; und X wie oben definiert ist.

2. Plattierungsmedium nach Anspruch 1, wobei besagtes Me⁺ in den Formeln (I) und (IV) ein von Natriumhydroxid, Kaliumhydroxid, Lithiumhydroxid, Ammoniumhydroxid, Diethylamin, Triethylamin, Tetramethylammoniumhydroxid, Tetraethylammoniumhydroxid, Cyclohexylamin, Pyridin, Piperidin, Pyrrolidin, Morpholin, N-Methyl-morpholin, N-Ethyl-morpholin und p-Toluidin abgeleitetes Kation ist.

3. Plattierungsmedium nach Anspruch 1 oder 2, wobei ein jeder der besagten fluorogenen Substituenten in Formel (I) unabhängig ausgewählt ist aus der Gruppe bestehend aus C₁-C₄-Alkyl, das gegebenenfalls in der Alkylkette ein Sauerstoffatom enthält; C₁-C₄-Alkoxy; Nitro; Carboxy, C₁-C₄-Carboxyalkyl und Cyano, wobei eine jede der besagten Alkylgruppen gegebenenfalls mindestens ein Halogenatom als Substituent aufweist.

4. Plattierungsmedium nach Anspruch 3, wobei R³ in Formel (I) ein C₁-C₄-Alkyl ist, das gegebenenfalls ein oder mehrere Halogenatome enthält, X Hydroxyl ist, und R¹, R², R⁴ und R⁵ Wasserstoffatome sind; und wobei besagtes Salz von besagter Verbindung der Formel (I) ein mit einer organischen oder anorganischen Base gebildetes Salz ist.

5. Plattierungsmedium nach irgend einem der Ansprüche 1 bis 4, wobei besagte Verbindung der Formel (I) ausgewählt ist aus 4-Methylumbelliferylmyo-inositol-1-phosphat und dessen Salzen mit einer organischen oder anorganischen Base.

6. Plattierungsmedium nach irgend einem der Ansprüche 1 bis 5, wobei in besagter Formel (IV) R⁶, R⁷ und R⁸ ausgewählt sind aus Wasserstoff, Chlor und Brom; R⁹ und R¹⁰ Wasserstoff sind und/oder wobei besagte Verbindung der Formel (IV) in der Form eines Salzes mit einer organischen oder anorganischen Base vorliegt.

7. Plattierungsmedium nach irgend einem der Ansprüche 1 bis 6, wobei besagte Verbindung der Formel (IV) ausgewählt ist aus 5-Bromo-4-chloro-3-indoxylmyoinositol-1-phosphat, 5-Bromo-6-chloro-3-indoxyl-myo-inositol-1-phosphat, 6-chloro-3-indoxyl-myo-inositol-1-phosphat und einem Salz davon.

8. Plattierungsmedium nach Anspruch 7, wobei besagtes Salz ein Ammoniumsalz der besagten Verbindung der Formel (IV) ist.

9. Plattierungsmedium nach irgend einem der Ansprüche 1 bis 8, zusätzlich enthaltend mindestens einen gelbildenden Bestandteil und mindestens einen zur Aufrechterhaltung des Wachstums von besagtem Mikroorganismus fähigen Nährstoff.

10. Plattierungsmedium nach irgend einem der Ansprüche 1 bis 9, zusätzlich enthaltend mindestens einen Inhibitor für andere Mikroorganismen als besagter interessierender Mikroorganismus.

11. Plattierungsmedium nach Anspruch 9 oder 10, wobei besagter Nährstoff ausgewählt ist aus der Gruppe bestehend aus Peptonen und Aminosäuren.

12. Plattierungsmedium nach Anspruch 11, wobei besagte Aminosäuren als sauer hydrolysiertes Kasein in einer Menge von ungefähr 2 bis ungefähr 20 Gramm pro Liter von besagtem Medium mit Zugabe von L-Cystein und L-Tryptophan in einer Menge von ungefähr 10 bis ungefähr 200 Milligramm pro Liter verwendet werden.

13. Plattierungsmedium nach irgend einem der Ansprüche 9 bis 12, wobei besagte Nährstoffe mittels Nährstoffanalyse ausgewählt sind und wobei besagter Nährstoff mindestens ein Mitglied der Gruppe bestehend aus Hefenextrakt, Fleischextrakt und Brain-Heart-Infusion in einer Menge von ungefähr 1 bis ungefähr 12 Gramm pro Liter von besagtem Medium umfasst.

14. Plattierungsmedium nach irgend einem der Ansprüche 1 bis 13, zusätzlich umfassend mindestens ein Kohlenhydrat ausgewählt aus der Gruppe bestehend aus D-Glukose, Natriumpyruvat und L-Rhamnose in einer Menge von ungefähr 0.5 bis ungefähr 5 Gramm pro Liter von besagtem Medium.

15. Plattierungsmedium nach irgend einem der Ansprüche 1 bis 14, zusätzlich umfassend Spurenelemente, die zur Verbesserung des Wachstums von besagtem Mikroorganismus fähig sind.

16. Plattierungsmedium nach irgend einem der Ansprüche 1 bis 15, zusätzlich umfassend mindestens eine Eisen(III)-Verbindung ausgewählt aus der Gruppe von Eisen(III)-Zitrat, Eisen(III)-Ammoniumzitrat und Ferrioxaminen.

17. Plattierungsmedium nach irgend einem der Ansprüche 1 bis 16, zusätzlich umfassend einen Promoter zur Spaltung von PI-PLC ausgewählt aus der Gruppe bestehend aus Albuminen, Phospholipoiden und Glycerophosphorsäuren.

18. Plattierungsmedium nach irgend einem der Ansprüche 1 bis 17, zusätzlich umfassend einen Induktor für die Enzymproduktion, z.B. Lecithin, wobei die Spaltung von besagtem Induktor durch Verbesserung der Färbung der Kolonie und durch Bildung einer weissen Niederschlagszone, welche die fluoreszierenden und/oder angefärbten Kolonien umgibt, zu einer besseren Erkennung des Zielmikroorganismus beiträgt.

19. Plattierungsmedium nach irgend einem der Ansprüche 1 bis 18, zusätzlich umfassend ein kontrastverstärkendes Agens zur Verbesserung der visuellen Nachweisbarkeit von gefärbten Kolonien des besagten interessierenden Mikroorganismus.

20. Plattierungsmedium nach irgend einem der Ansprüche 10 bis 19, wobei besagter Inhibitor ausgewählt ist aus der Gruppe bestehend aus Verbindungen, die zur Inhibierung des Wachstums von gram-negativen Bakterien, gram-positiven Bakterien, Hefen und Pilzen fähig sind.

21. Plattierungsmedium nach irgend einem der Ansprüche 10 bis 20, wobei besagter Inhibitor ausgewählt ist aus Polymyxin, Sulfamethazol, Sulfamethoxazol, Phosphomycin, Doxycyclin, Ceftazidim, Clindamycin, Nalidixinsäure, Cycloheximid, Trimethoprim und Amphotericin.

22. Plattierungsmedium nach irgend einem der Ansprüche 1 bis 21 zur Verwendung in einem Direktkontaktverfahren durch direkten Kontakt mit einer Probe mit Verdacht, den besagten interessierenden Mikroorganismus zu enthalten, umfassend mindestens einen Bestandteil zur Entgegenwirkung mit oberflächenaktiven Komponenten einer Probe.

23. Plattierungsmedium nach Anspruch 22, wobei besagter Bestandteil zur Entgegenwirkung mit oberflächenaktiven Komponenten einer Probe Lecithin und L-Histidin umfasst.

24. Plattierungsmedium nach irgend einem der Ansprüche 1 bis 23, enthaltend ein wässriges Medium zum Bereitstellen eines gebrauchsfertigen Plattierungsmediums.

25. Plattierungsmedium nach irgend einem der Ansprüche 1 bis 23 in einer im Wesentlichen wasserfreien Form.

26. Plattierungsmedium nach irgend einem der Ansprüche 1 bis 25, wobei ausgewählte Antibiotika-Scheiben auf die Oberfläche dieses Plattierungsmediums zur weiteren Differenzierung der wachsenden Bakterien aufgrund von deren unterschiedlichen Resistenzen gebracht werden.

27. Plattierungsmedium nach irgend einem der Ansprüche 1 bis 26, wobei besagter nachzuweisender Mikroorganismus ausgewählt ist aus *Listeria monocytogenes, Listeria ivanovii, Bacillus cereus,* probiotschen Strängen von *Bacillus cereus, Bacillus thuringiensis, Bacillus mycoides, Bacillus anthracis, Staphylococcus aureus, Legionella pneumophila, Clostridium species, Helicobacter pylori, Candida species;* und *Aspergillus species.*

28. Verfahren zum Nachweis eines interessierenden Mikroorganismus, wobei besagter Mikroorganismus vom Typ ist, der zur metabolischen Produktion einer phosphatidylinositol-spezifischen Phospholipase C (PI-PLC) fähig ist; umfassend die Schritte:
(a) Bereitstellen eines Plattierungsmediums enthaltend ein wässriges Gel und mindestens einen Nährstoff, der zur Aufrechterhaltung des Wachstums von besagtem Mikroorganismus fähig ist; und mindestens eine Verbindung der Formel (I) wie in Anspruch 1 definiert, und mindestens eine Verbindung der Formel (IV) wie in Anspruch 1 definiert;
(b) Inokulieren des besagten Plattierungsmediums mit einem Probenmaterial mit dem Verdacht, den besagten interessierenden Mikroorganismus zu enthalten;
(c) Inkubieren des besagten Plattierungsmediums im Anschluss an besagten Schritt (b);
(d) gegebenenfalls Anbringen von ausgewählten Antibiotika-Scheiben auf die Oberfläche des Plattierungsmediums zur weiteren Differenzierung von wachsenden Bakterien aufgrund ihrer unterschiedlichen Resistenz; und
(e) Beobachten des besagten Plattierungsmediums im Anschluss an besagten Schritt (c) oder (d) bezüglich der Bildung von fluoreszierenden und/oder angefärbten Kolonien von besagtem interessierendem Mikroorganismus als Nachweis für die Anwesenheit von besagtem Mikroorganismus in der besagten Probe.

29. Verfahren nach Anspruch 28, wobei besagte Verbindung der Formel (I) 4-Methylumbelliferyl-myo-inositol-1-phosphat oder ein Salz davon ist.

30. Verfahren nach Anspruch 28 oder 29, wobei besagte Verbindung der Formel (IV) 5-Bromo-4-chloro-3-indoxylmyo-inositol-1-phosphat oder ein Salz davon ist.

31. Kombination von mindestens einer Verbindung der Formel (I) wie in Anspruch 1 definiert und mindestens einer Verbindung der Formel (IV) wie in Anspruch 1 definiert, zur Verwendung für den Nachweis von Mikroorganismen des Typs, der zur metabolischen Produktion einer phosphatidylinositol-spezifischen Phospholipase C (PI-PLC) fähig ist.

## Revendications

1. Milieu de mise en culture pour la détection d'un microorganisme du type capable de production métabolique d'une phospholipase C spécifique au phosphatidylinositol (PI-PLC),
**caractérisé en ce que**
le milieu de mise en culture contient, en combinaison, au moins un composé fluorogénique capable de générer de la fluorescence lorsqu'il est en contact avec la PI-PLC, et au moins un composé chromogène capable de produire une couleur lorsqu'il est en contact avec la PI-PLC ; dans lequel le composé fluorogénique est représenté par la formule (I) 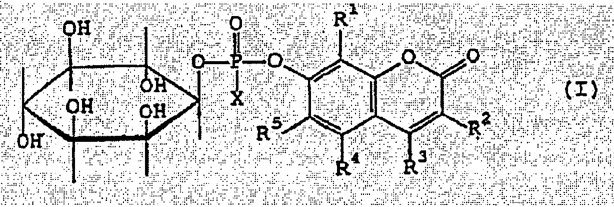 dans laquelle R¹, R², R³, R⁴ et R⁵ sont indépendamment sélectionnés dans le groupe constitué de l'hydrogène et de substituants fluorogéniques, et X est sélectionné dans le groupe constitué de : un hydroxyle ; OR^{y}, dans lequel R^{y} est sélectionné dans le groupe constitué d'un alkyle en C₁-C₄ ; et O-Me⁺, dans lequel Me⁺ est un cation dérivé d'une base organique ou minérale,
et dans lequel le composé chromogène est représenté par la formule (IV) 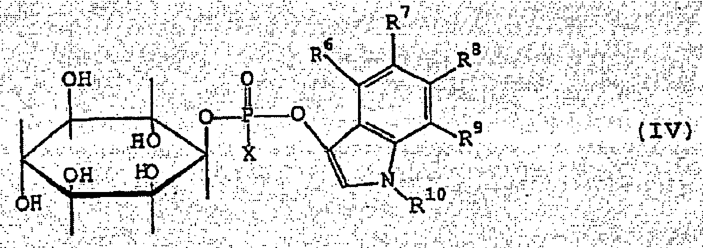 dans laquelle R¹⁰ est sélectionné dans le groupe constitué de l'hydrogène et d'un alkyle en C₁₋₄ et R⁶, R⁷, R⁸ et R⁹ sont sélectionnés dans le groupe constitué de l'hydrogène et de substituants chromogènes ; et X est tel que défini ci-dessus.

2. Milieu de mise en culture selon la revendication 1,
**caractérisé en ce que**
le Me⁺ dans la formule (I) et (IV) est un cation dérivé d'hydroxyde de sodium, d'hydroxyde de potassium, d'hydroxyde de lithium, d'hydroxyde d'ammonium, de diéthylamine, de triéthylamine, d'hydroxyde de tétraméthylammonium, d'hydroxyde de tétraéthylammonium, de cyclohexylamine, de pyridine, de pipéridine, de pyrrolidine, de morpholine, de N-méthyl-morpholine, de N-éthyl-morpholine et de p-toluidine.

3. Milieu de mise en culture selon la revendication 1 ou 2,
**caractérisé en ce que**
chacun des substituants fluorogéniques dans la formule (I) est indépendamment sélectionné dans le groupe constitué d'un alkyle en C₁-C₄ contenant en option un atome d'oxygène dans la chaîne alkyle ; un alkoxy en C₁-C₄ ; un nitro ; un carboxy, un carboxyalkyle en C₁-C₄ et un cyano dans lequel l'un quelconque des groupes alkyle comprend en option au moins un atome d'halogène comme substituant.

4. Milieu de mise en culture selon la revendication 3,
**caractérisé en ce que**
R³ dans la formule (I) est un alkyle en C₁-C₄ contenant en option un ou plusieurs atomes d'halogène, X est hydroxyle et R¹, R², R⁴ et R⁵ sont des atomes d'hydrogène ; et le sel du composé de formule (I) est un sel formé avec une base organique ou minérale.

5. Milieu de mise en culture selon l'une quelconque des revendications 1 à 4,
**caractérisé en ce que**
le composé de formule (I) est sélectionné parmi le myo-inositol-1-phosphate de 4-méthylumbelliféryle et des sels de celui-ci avec une base organique ou minérale.

6. Milieu de mise en culture selon l'une quelconque des revendications
1 à 5,
**caractérisé en ce que**
R⁶, R⁷ et R⁸ dans la formule (IV) sont sélectionnés parmi l'hydrogène, le chlore et le brome ; R⁹ et R¹⁰ sont de l'hydrogène et/ou le composé de formule (IV) est sous la forme d'un sel avec une base organique ou minérale.

7. Milieu de mise en culture selon l'une quelconque des revendications 1 à 6,
**caractérisé en ce que**
le composé de formule (IV) est sélectionné parmi le myo-inositol-1-phosphate de 5-bromo-4-chloro-3-indoxyle, le myo-inositol-1-phosphate de 5-bromo-6-chloro-3-indoxyle, le myo-inositol-1-phosphate de 6-chloro-3-indoxyle et un sel de celui-ci.

8. Milieu de mise en culture selon la revendication 7,
**caractérisé en ce que**
le sel est un sel d'ammonium du composé de formule (IV).

9. Milieu de mise en culture selon l'une quelconque des revendications 1 à 8, contenant en outre au moins un constituant gélifiant et au moins un nutriment capable de supporter la croissance du microorganisme.

10. Milieu de mise en culture selon l'une quelconque des revendications 1 à 9, contenant en outre au moins un inhibiteur pour des microorganismes autres que le microorganisme pertinent.

11. Milieu de mise en culture selon la revendication 9 ou 10,
**caractérisé en ce que**
le nutriment est sélectionné dans le groupe constitué des peptones et des acides aminés.

12. Milieu de mise en culture selon la revendication 11,
**caractérisé en ce que**
les acides aminés sont utilisés comme caséine hydrolysée par voie acide en une quantité allant d'environ 2 à environ 20 grammes par litre du milieu avec addition de L-cystéine et de L-tryptophane en une quantité allant d'environ 10 à environ 200 milligrammes par litre.

13. Milieu de mise en culture selon l'une quelconque des revendications 9 à 12, dans lequel les nutriments sont sélectionnés par analyse de nutriments, et le nutriment comprend au moins un membre du groupe constitué d'un extrait de levure, d'un extrait de viande et d'un milieu coeur-cervelle en une quantité allant d'environ 1 à environ 12 grammes par litre du milieu.

14. Milieu de mise en culture selon l'une quelconque des revendications 1 à 13, comprenant en outre au moins un glucide sélectionné dans le groupe constitué du D-glucose, du pyruvate de sodium et du L-rhamnose en une quantité allant d'environ 0,5 à environ 5 grammes par 1 du milieu.

15. Milieu de mise en culture selon l'une quelconque des revendications 1 à 14, comprenant en outre des oligoéléments capables d'améliorer la croissance du microorganisme.

16. Milieu de mise en culture selon l'une quelconque des revendications 1 à 15, comprenant en outre au moins un composé ferrique sélectionné dans le groupe du citrate ferrique, du citrate d'ammonium ferrique et des ferrioxamines.

17. Milieu de mise en culture selon l'une quelconque des revendications 1 à 16, comprenant en outre un promoteur pour le clivage de PI-PLC sélectionné dans le groupe constitué des albumines, des phospholipoïdes et des acides glycérophosphoriques.

18. Milieu de mise en culture selon l'une quelconque des revendications 1 à 17, comprenant en outre un inducteur pour la production enzymatique, par exemple, la lécithine, dans lequel le clivage de l'inducteur contribue à une meilleure reconnaissance du microorganisme cible par amélioration de la coloration de colonie et par formation d'une zone de précipitation blanche entourant les colonies fluorescentes et/ou colorées.

19. Milieu de mise en culture selon l'une quelconque des revendications 1 à 18, comprenant en outre un produit de contraste pour améliorer la capacité de détection visuelle de colonies teintées du microorganisme pertinent.

20. Milieu de mise en culture selon l'une quelconque des revendications 10 à 19,
**caractérisé en ce que**
l'inhibiteur est sélectionné dans le groupe constitué de composés capables d'inhiber la croissance de bactéries Gram-négatives, de bactéries Gram-positives, de levures et de champignons.

21. Milieu de mise en culture selon l'une quelconque des revendications 10 à 20,
**caractérisé en ce que**
l'inhibiteur est sélectionné parmi la polymyxine, le sulfaméthazole, le sulfaméthoxazole, la phosphomycine, la doxycycline, la ceftazidime, la clindamycine, l'acide nalidixique, le cycloheximide, la triméthoprime et l'amphotéricine.

22. Milieu de mise en culture selon l'une quelconque des revendications 1 à 21, pour une utilisation dans un procédé de contact direct par contact direct avec un échantillon susceptible de contenir le microorganisme pertinent comprenant au moins un constituant pour contrecarrer les composants tensioactifs d'un échantillon.

23. Milieu de mise en culture selon la revendication 22,
**caractérisé en ce que**
le constituant pour contrecarrer les composants tensioactifs d'un échantillon comprend la lécithine et la L-histidine.

24. Milieu de mise en culture selon l'une quelconque des revendications 1 à 23, contenant un milieu aqueux pour fournir un milieu de mise en culture prêt à l'emploi.

25. Milieu de mise en culture selon l'une quelconque des revendications 1 à 23, sous une forme essentiellement anhydre.

26. Milieu de mise en culture selon l'une quelconque des revendications 1 à 25, dans lequel des disques antibiotiques sélectionnés sont placés à la surface de ce milieu de mise en culture pour une différenciation supplémentaire des bactéries croissantes par leur résistante différente.

27. Milieu de mise en culture selon l'une quelconque des revendications 1 à 26,
**caractérisé en ce que**
le microorganisme devant être détecté est sélectionné parmi Listeria monocytogenes, Listeria ivanovii, Bacillus cereus, des souches probiotiques de Bacillus cereus, Bacillus thuringiensis, Bacillus mycoides, Bacillus anthracis, Staphylococcus aureus, Legionella pneumophila, des espèces de Clostridium, Helicobacter pylori, des espèces de Candida ; et des espèces de Aspergillus.

28. Procédé pour la détection d'un microorganisme pertinent, le microorganisme étant du type capable de production métabolique d'une phospholipase C spécifique au phosphatidylinositol (PI-PLC) ; comprenant les étapes consistant à :
(a) fournir un milieu de mise en culture contenant un gel aqueux et au moins un nutriment capable de supporter la croissance du microorganisme ; et au moins un composé de formule (I) tel que défini à la revendication 1 et au moins un composé de formule (IV) tel que défini à la revendication 1 ;
(b) inoculer le milieu de mise en culture avec un matériau échantillon susceptible de contenir le microorganisme pertinent ;
(c) incuber le milieu de mise en culture suite à l'étape (b) ;
(d) placer en option des disques antibiotiques sélectionnés à la surface de ce milieu de mise en culture pour une différenciation supplémentaire des bactéries croissantes par leur résistante différente ; et
(e) observer le milieu de mise en culture suite à la étape (c) ou (d) pour la formation de colonies fluorescentes et/ou colorées du microorganisme pertinent indicatrice d'une présence du microorganisme dans l'échantillon.

29. Procédé selon la revendication 28,
**caractérisé en ce que**
le composé de formule (I) est le myo-inositol-1-phosphate de 4-méthylumbelliféryle ou un sel de celui-ci.

30. Procédé selon la revendication 28 ou 29,
**caractérisé en ce que**
le composé de formule (IV) est le myo-inositol-1-phosphate de 5-bromo-4-chloro-3-indoxyle ou un sel de celui-ci.

31. Combinaison d'au moins un composé de formule (I) tel que défini à la revendication 1 et d'au moins un composé de formule (IV) tel que défini à la revendication 1, pour une utilisation dans la détection de microorganismes du type capable de production métabolique d'une phospholipase C spécifique au phosphatidylinositol (PI-PLC).
